# EUROPÄISCHE PATENTSCHRIFT

(11) **EP 1 303 632 B1**
(45) Veröffentlichungstag und Bekanntmachung des Hinweises auf die Patenterteilung: **06.10.2004**
(21) Anmeldenummer: 00991171.0
(22) Anmeldetag: 12.12.2000
(51) Int. Cl.: C12P 19/14, C12N 9/42, C12N 11/00, C07H 3/04, C07H 3/06, C08B 37/00, A23L 1/308, A61K 7/48

(54) **GALACTOMANNAN-OLIGOSACCHARIDE UND VERFAHREN ZU DEREN HERSTELLUNG SOWIE DEREN VERWENDUNG**
GALACTOMANNAN OLIGOSACCHARIDES AND METHODS FOR THE PRODUCTION AND USE THEREOF
OLIGOSACCHARIDES DE GLACTOMANNANE, PROCEDES DE FABRICATION ET UTILISATIONS DE CES COMPOSES

(30) Priorität: 18.12.1999 DE 19961182
(43) Veröffentlichungstag der Anmeldung: 23.04.2003
(73) Patentinhaber: SÜDZUCKER AKTIENGESELLSCHAFT, 68165 Mannheim (DE)
(72) Erfinder: KUNZ, Markwart, 67550 Worms (DE); VOGEL, Manfred, 67271 Neuleiningen (DE); KLINGEBERG, Michael, 67269 Grünstadt (DE); LUDWIG, Eva, 69181 Leimen (DE); MUNIR, Mohammad, 67271 Kindenheim (DE); RITTIG, Frank, 67269 Grünstadt (DE)
(74) Vertreter: Schrell, Andreas, Dr.
(86) Internationale Anmeldenummer: PCT/EP2000/012574
(87) Internationale Veröffentlichungsnummer: WO 2001/044489

(56) Entgegenhaltungen:
- US-A- 3 684 710
- DATABASE BIOSIS [Online] BIOSCIENCES INFORMATION SERVICE, PHILADELPHIA, PA, US; Februar 1999 (1999-02) CUI FUMIAN ET AL: "Production of neutral bata-mannanase by Bacillus subtilis and its properties." Database accession no. PREV199900323995 XP002176485 & WEISHENGWU XUEBAO, Bd. 39, Nr. 1, Februar 1999 (1999-02), Seiten 60-63, ISSN: 0001-6209
- PATENT ABSTRACTS OF JAPAN vol. 018, no. 001 (C-1148), 6. Januar 1994 (1994-01-06) & JP 05 246859 A (TAIYO KAGAKU CO LTD), 24. September 1993 (1993-09-24)
- DATABASE BIOSIS [Online] BIOSCIENCES INFORMATION SERVICE, PHILADELPHIA, PA, US; 1989 VLASENKO E YU ET AL: "HYDROLYSIS OF MANNOSE-CONTAINING POLYSACCHARIDES BY ENDO-1 4-BETA-GLYCANASES" Database accession no. PREV199089069702 XP002176486 & PRIKLADNAYA BIOKHIMIYA I MIKROBIOLOGIYA, Bd. 25, Nr. 5, 1989, Seiten 604-613, ISSN: 0555-1099
- PATENT ABSTRACTS OF JAPAN vol. 012, no. 275 (C-516), 29. Juli 1988 (1988-07-29) & JP 63 056289 A (RES DEV CORP OF JAPAN;OTHERS: 03), 10. März 1988 (1988-03-10)
- PATENT ABSTRACTS OF JAPAN vol. 006, no. 139 (C-116), 28. Juli 1982 (1982-07-28) & JP 57 065182 A (AGENCY OF IND SCIENCE & TECHNOL), 20. April 1982 (1982-04-20)
- RATTO M ET AL: "PRODUCTION OF MANNAN-DEGRADING ENZYMES" BIOTECHNOLOGY LETTERS, Bd. 10, Nr. 9, 1988, Seiten 661-664, XP001015892 ISSN: 0141-5492
- MCCLEARY B V ET AL: "CHARACTERISATION OF THE OLIGOSACCHARIDES PRODUCED ON HYDROLYSIS OF GALACTOMANNAN WITH BETA-D-MANNANASE" CARBOHYDRATE RESEARCH, ELSEVIER SCIENTIFIC PUBLISHING COMPANY. AMSTERDAM, NL, Bd. 118, 1983, Seiten 91-109, XP000891369 ISSN: 0008-6215
- PATENT ABSTRACTS OF JAPAN vol. 1996, no. 08, 30. August 1996 (1996-08-30) & JP 08 099884 A (TAIYO KAGAKU CO LTD), 16. April 1996 (1996-04-16)
- DAVIS A L ET AL: "H- and C-NMR characterization of the digalactosylmannopentaose liberated from legume seed galactomannan by beta-mannanase action" CARBOHYDRATE RESEARCH, ELSEVIER SCIENTIFIC PUBLISHING COMPANY. AMSTERDAM, NL, Bd. 271, Nr. 1, 10. Juli 1995 (1995-07-10), Seiten 43-54, XP004022002 ISSN: 0008-6215

## Beschreibung

Die vorliegende Erfindung betrifft Galacto-Manno-Oligosaccharide, Verfahren zu deren Herstellung sowie ihre Verwendung in Lebensmitteln und Arzneimitteln.

Es besteht ständig ein Bedarf, aus nachwachsenden natürlichen Rohstoffen Wirk- und/oder Zusatzstoffe für Lebens- und/oder Arzneimittel zu gewinnen. Dies liegt sowohl an der hohen Akzeptanz aus natürlichen Quellen gewonnener Rohstoffe beim Verbraucher als auch an der Umweltverträglichkeit ihrer Herstellung. Mannane und deren Derivate, wie Glucomannan und Galactomannan, stellen derartige natürliche Rohstoffe dar. Mannane sind Polyosen, die aus Mannose anstelle von Glucoseeinheiten aufgebaut sind. Die Mannose-Ketten bestehen aus β-1,4-verknüpften Mannose-Einheiten. Galactomannane weisen neben den β-1,4-verknüpften Mannose-Einheiten damit α-1,6-verknüpfte Galactose-Einheiten auf, bestehen also aus Mannose- und Galactosebausteinen. Derartige Galactomannane sind in Form von Guargum, Cassiagum und Johannisbrot-Kernmehl erhältlich und werden zum Beispiel als Verdickungsmittel in der Lebensmittelindustrie und als Tablettierhilfsmittel in der Pharmaindustrie verwendet (Industrial Gums, R. L. Whistler und J.N. BeMiller, Herausgeber, 3. Auflage 1992, Academic Press, New York). Galactomannane aus verschiedenen Quellen unterscheiden sich im wesentlichen durch ihren relativen Mannose- und Galactose-Gehalt.

Die Verwendung von Mannanasen aus Bacillus subtilis zur Hydrolyse von Mannose-haltigen Polysacchariden zu einem Gemisch aus Mannose- und Galactose-haltigen Oligosacchariden ist bekannt. In Vlasenko et al. (Prikladnaya Biokhimiya i Mikrobiologiya, 1989, 25(5):604-613) ist die Hydrolyse von Johannisbrotkernmehl mit einer Mannanase aus Bacillus subtilis beschrieben, wobei Oligosaccharide mit einem Polymerisationsgrad >3 erhalten werden. R 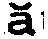 ttö M. et al. (Biotechnology Letters, 1988, 10 (9):661-664) beschreiben die Hydrolyse von Johannisbrotkernmehl und Guargummi, wobei Guargummi mit der dort eingesetzten Mannanase aus Bacillus subtilis nur zu einem sehr geringen Teil hydrolysiert wird. Cui Fumian et al. (Weishengwu Xuebato, 39 (1):60-63) beschreiben die Hydrolyse von Konjakmehl und Johannisbrotkernmehl mittels einer Mannanase aus dem Bacillus subtilis-Stamm BM 9602. In der JP 57 065182 A wird die Gewinnung von β-1,4-Mannanase aus dem Bacillus subtilis-Stämmen LB5A und LB5B beschrieben, wobei Galactomannane dem Kulturmedium hinzugefügt sind. Die US 3,684,710 A beschreibt die Hydrolyse von Johannisbrotkernmehl mittels eines Gemisches von Enzymen aus Bacillus subtilis und Aspergillus niger.

Zur Herstellung lebensmitteltauglicher Rohstoffe werden Polyosen häufig in kleinere Einheiten zerlegt. So ist es bekannt, Polysaccharide mit Hilfe von verdünnten Säuren bei unterschiedlichen Temperaturen zu hydrolysieren. Die Hydrolyse von Galactomannanen führt zu einem Gemisch der Monomere, also Mannose und Galactose.

Eine aus Aspergillus niger gewonnene endo-β-Mannanase (E.C. 3.2.1.78) ist bekannt dafür, Galactomannan enzymatisch zu hydrolysieren (H. Uhlig, Enzyme arbeiten für uns, Carl Hanser Verlag München, Wien, 1991). Dieses Enzym ist jedoch nicht in der Lage, das Galactomannan aus Guargum zu hydrolysieren, während Galactomannan aus Johannisbrot-Kernmehl nur partiell hydrolysiert wird. Je nach Herkunft können Galactomannane also eine unterschiedliche Eignung für eine Hydrolyse aufweisen.

Aus Ajisaka et al. (Carbohydrate Research 270 (1995), 123-130) ist ein Verfahren bekannt, das die enzymatische Synthese von Mannobiose und Mannotriose mit Hilfe einer aus Aspergillus niger isolierten α-Mannosidase erlaubt. Diese Synthese, die durch eine Umkehrung der normalen Hydrolysereaktion des Enzyms durchgeführt wird, arbeitet mit technisch nicht akzeptablen Ausbeuten von um 2 %. Die Produkte enthalten keine Galactose-Einheiten.

Aus K. Newman (Biotechnology in the Feed Industry, Proc. Of Alltech's 10^{th} Symposium (1994), 167-174) ist ein aus Hefe-Zellwand gewonnener Gluco-Manno-Proteinkomplex bekannt, der spezifisch die mannosespezifischen Lektine von pathogenen Keimen bindet. Da dieses Produkt keine Galactoseeinheiten enthält, kann es keine Bindung mit galactosespezifischen mikrobiellen Lektinen vermitteln.

Das der vorliegenden Erfindung zu Grunde liegende technische Problem besteht also darin, aus Galactomannan herstellbare Substanzen sowie Verfahren zu deren Herstellung bereitzustellen, die vorteilhafte Anwendungsmöglichkeiten im Bereich der Pharmazie und Lebensmitteltechnologie mit sich bringen.

Das der vorliegenden Erfindung zu Grunde liegende technische Problem wird durch die Bereitstellung eines Verfahrens zur Herstellung von Mannose- und Galactose-haltigen Oligosacchariden aus Galactomannanen gelöst, wobei eine wässrige Lösung oder Suspension des Galactomannans hergestellt, durch aus Bakterien stammende enzymatische Aktivität, insbesondere unter Einsatz eines aus Bakterien stammenden enzymatischen Agens, hydrolysiert und ein Gemisch aus Mannose- und Galactose-haltigen Oligosacchariden mit einem Polymerisationsgrad (DP) < 15, insbesondere von 2 bis 7, erhalten wird. Die Erfindung löst das ihr zu Grunde liegende Problem auch durch die Bereitstellung derartig herstellbarer Galacto-Manno-Oligosaccharide, umfassend β-1,4-verknüpfte Mannose-Einheiten und daran α-1,6-verknüpfte Galactose-Einheiten mit einem Polymerisationsgrad < 15, insbesondere von 2 bis 7. Diese Galacto-Manno-Oligosaccharide zeichnen sich in überraschender und vorteilhafter Weise dadurch aus, dass sie bei Verwendung in oder als Nahrungs-, Lebens-, Genuss- und Arzneimitteln die Prophylaxe und Therapie von Krankheiten ermöglichen sowie allgemein zur Verbesserung des Gesundheitszustandes dienen können.

Insbesondere zeichnen sich die erfindungsgemäßen Galacto-Manno-Oligosaccharide dadurch aus, dass sie den glykämischen Index von Genuss- und Lebensmitteln senken, Infektionskrankheiten bekämpfen und/oder verhindern, indem sie die Anlagerung von pathogenen Keimen an menschliche und tierische Epithelzellen verhindern oder reduzieren, entzündliche chronische Darmerkrankungen bekämpfen und/oder verhindern, der Entstehung von Darmkrebs beziehungsweise Coloncarzinomen entgegenwirken und/oder diese bekämpfen, die Immunabwehr gegen allgemeine Infekte stärken, die Immunabwehr modulieren und damit entzündliche Erkrankungen bekämpfen und/oder verhindern und die Calciumaufnahme verbessern und dadurch insbesondere Osteoporose entgegenwirken.

Im Zusammenhang mit der vorliegenden Erfindung wird unter einer Krankheit eine Störung der Lebensvorgänge in Organen oder im gesamten Organismus verstanden, die subjektiv empfundene oder objektiv feststellbare körperliche, geistige oder seelische Veränderungen mit sich bringt. Im Zusammenhang mit der vorliegenden Erfindung werden unter einer Krankheit auch Mangelzustände erfasst.

Im Zusammenhang mit der vorliegenden Erfindung wird unter einem Wirkstoff eine Substanz verstanden, die in lebenden Organismen oder Teilen davon eine biologische Wirkung hervorruft. Unter einem Arzneistoff wird ein Wirkstoff verstanden, der zur Vorbeugung, Linderung, Heilung oder Erkennung von Erkrankungen dienen kann. Unter einem Arzneimittel wird eine zur Anwendung bei Menschen oder Tieren bestimmte Zubereitungsform von Arzneistoffen verstanden.

Im Zusammenhang mit der vorliegenden Erfindung wird unter einem Nahrungs- oder Lebensmittel ein primär der Aufrechterhaltung der Lebensfunktionen dienendes Mittel verstanden, während ein Genussmittel ein primär dem bei seiner Aufnahme entstehenden Wohlbefinden dienendes Mittel verstanden wird.

Die vorliegende Erfindung betrifft also Mannoseund Galactose-haltige Oligosaccharide, auch als Galacto-Manno-Oligosaccharide bezeichnet, mit einem Polymerisationsgrad (DP) < 15, insbesondere mit einem Polymerisationsgrad von 2 bis 7, wobei die Mannose-Einheiten β-1,4 und die Galactose-Einheiten mit den Mannose-Einheiten α-1,6 verknüpft sind. Die erfindungsgemäßen Galacto-Manno-Oligosaccharide sind gegenüber den hydrolytischen Bedingungen im Mund-, Magen- und Dünndarmbereich stabil und können somit im wesentlichen unverändert in den Dickdarm gelangen und dort ihre gewünschte gesundheitsfördernde Wirkung entfalten.

Die erfindungsgemäßen Galacto-Manno-Oligosaccharide sind nicht nur selbst unter den hydrolytischen Bedingungen im Dünndarm stabil, sondern sie inhibieren sogar die mucosaständigen α-Glucosidasen (Glucoamylase/Maltase und Saccharase/Isomaltase). Sie können daher erfindungsgemäß zum Senken des glykämischen Index von Genuss- und Lebensmitteln eingesetzt werden.

Erfindungsgemäß sind die Galacto-Manno-Oligosaccharide der vorliegenden Erfindung auch in der Lage, die Anheftung von pathogenen Keimen an Epithelzellen zu reduzieren oder zu verhindern und dadurch Infektionskrankheiten vorzubeugen. sowie in ihrem Verlauf zu therapieren. Die erfindungsgemäßen Galacto-Manno-Oligosaccharide stimulieren zudem die Mucussekretion aus den Becherzellen im Darm und beeinflussen daher den Verlauf verschiedener Darmerkrankungen positiv.

Wie erwähnt, werden die erfindungsgemäßen Galacto-Manno-Oligosaccharide im Dünndarm nicht hydrolysiert, sondern gelangen unverändert in den Dickdarm, wo sie dann von den dort vorhandenen Mikroorganismen zu kurzkettigen Fettsäuren, insbesondere Butyrat, fermentiert werden. Die infolge dieser Fermentation erfolgende pH-Absenkung verschlechtert die Lebensbedingungen für schädliche Mikroorganismen wie Clostridien und verbessert gleichzeitig die Lebensbedingungen für nützliche Bifidobakterien und Lactobacillen. Die erfindungsgemäßen Galacto-Manno-Oligosaccharide weisen also präbiotische Wirkung auf. Durch die beschriebene vermehrte Bildung von Butyrat kann die Entstehung und das Wachstum von Coloncarzinomen reduziert beziehungsweise verhindert werden.

Schließlich sind die erfindungsgemäßen Galacto-Manno-Oligosaccharide vorteilhaft insofern, als dass sie die Aufnahme von Calcium im Darmbereich aus anorganischen Nahrungsbestandteilen verbessern und so insbesondere bei der Vorbeugung und Verhinderung von Osteoporose, insbesondere primärer Osteoporose, wie postmenopausaler oder seniler Osteoporose oder sekundärer Osteoporose, einsetzbar sind.

Die erfindungsgemäßen Galacto-Manno-Oligosaccharide sind schließlich auch insofern vorteilhaft einsetzbar, als dass sie direkte Wechselwirkungen mit dem zellulären Immunsystem zeigen, wobei sie einerseits die Immunabwehr stärken und andererseits die Immunantwort so modulieren, dass entzündliche Prozesse abgemindert beziehungsweise unterdrückt werden.

Die Erfindung betrifft daher auch Genuss-, Lebensoder Nahrungsmittel sowie sogenannte Functional Foods, die die erfindungsgemäßen Galacto-Manno-Oligosaccharide enthalten. Derartige Lebensmittel können zum Beispiel Milchprodukte wie Butter, Joghurt, Quark, Backwaren, Suppen- und Sossenpulver, Brotaufstriche, Margarine, Backfette, Gewürzmischungen, Konfitüren, nichtalkoholische Getränke etc. sein. Genussmittel können zum Beispiel Hartoder Weichkaramellen, Kaugummis, Schokolade, Müsliriegel, Keksprodukte, Eis, Schaumzuckerprodukte, Dragees, alkoholische und nichtalkoholische Getränke etc. sein.

Die Erfindung betrifft auch Arzneimittel, die die erfindungsgemäßen Galacto-Manno-Oligosaccharide, gegebenenfalls zusammen mit pharmakologisch geeigneten Träger-, Zusatz- oder Hilfsstoffen, in einer pharmazeutisch wirksamen Menge enthalten. Derartige Träger-, Hilfs- oder Zusatzstoffe können zum Beispiel Gleitmittel, Trennmittel, Verdickungsmittel, Stabilisatoren, Emulgatoren, Konservierungsstoffe, Lecithin, Intensivsüßstoffe, Süßungsmittel, Farbstoffe, Geschmacksstoffe, Aromastoffe, bulking agents, also Füllstoffe, etc. sein.

Die Arneimittel können zum Beispiel in Form von Pastillen, Kapseln, Tabletten, Dragees, Zäpfchen, Lösungen, Suspensionen, Emulsionen, Injektionslösungen, Infusionslösungen, Tropfen, Säften, Salben, Cremes, Gele, Aerosol, Inhalat oder anderen üblichen Darreichungsformen vorliegen.

Die Erfindung betrifft auch die erfindungsgemäßen Galacto-Manno-Oligosaccharide zur Anwendung in einem Verfahren zur chirurgischen oder therapeutischen Behandlung des menschlichen oder tierischen Körpers. Die Erfindung betrifft schließlich auch die Verwendung der erfindungsgemäßen Galacto-Manno-Oligosaccharide zur Prophylaxe oder Therapie von Diabetes mellitus II, Infektionserkrankungen, Darmerkrankungen, Coloncarzinomen, entzündlichen Erkrankungen und Osteoporose sowie die Verwendung der erfindungsgemäßen Galacto-Manno-Oligosaccharide zur Herstellung eines Arzneimittels für die vorgenannten Zwecke.

Die Erfindung betrifft auch Verfahren zur Herstellung der erfindungsgemäßen Galacto-Manno-Oligosaccharide, wobei eine wässrige Lösung oder Suspension eines Galactomannans aus einem Galactomannanhaltigen Rohstoff, insbesondere aus Guargum, zum Beispiel Guar-Mehl, Cassiagum oder Johannisbrot-Kernmehl hergestellt, unter Einsatz einer aus Bakterien stammenden enzymatischen Aktivität, insbesondere unter Einsatz eines aus Bakterien stammenden enzymatischen Agens, hydrolysiert und eine wässrige Lösung eines Gemischs aus Mannose- und Galactose-haltigen Oligosacchariden mit einem Polymerisationsgrad < 15, vorzugsweise von 2 bis 7, erhalten wird. Aus der wässrigen Produktlösung wird ein trockenes Gemisch des Produkts durch zum Beispiel Sprühtrocknung erhalten.

Die Erfindung stellt also die überraschende Lehre bereit, dass mittels einer aus Bakterien stammenden enzymatischen Aktivität Galactomannane aus diesen enthaltenden Rohstoffen, zum Beispiel aus Guargum, Cassiagum und Johannisbrot-Kernmehl, mit jeweils gleich hoher Effizienz zu den erfindungsgemäßen Galacto-Manno-Oligosacchariden hydrolysiert werden können.

Die Konzentration an Galactomannanen in der wässrigen Lösung beträgt vorzugsweise 1 bis 5 %, der pH-Wert vorzugsweise 5 bis 8 und die Temperatur der Lösung vorzugsweise 30 bis 40°C.

Im Zusammenhang mit der vorliegenden Erfindung wird unter einem enzymatisch wirkenden Agens ein vollständig oder teilweise aufgereinigtes Enzym oder ein Rohextrakt aus Bakterien, insbesondere Bacillus-Zellen oder lebende oder tote Bakterien verstanden, die Galactomannane hydrolysieren können, insbesondere zu Galacto-Manno-Oligosacchariden mit einem Polymerisationsgrad < 15, bevorzugt 2 bis 7. Rohextrakte können mittels üblicher Verfahren, wie mechanischer Aufschlussverfahren, zum Beispiel Kugelmühle, French Press, chemischer oder elektrischer Aufschlussverfahren, zum Beispiel Erzeugen elektrischer Felder oder auch durch Ultraschallbehandlung erhalten werden.

In besonders bevorzugter Ausführungsform der vorliegenden Erfindung werden Bakterien der Art Bacillus subtilis, insbesondere ein Bacillus-subtilis-Stamm mit der Hinterlegungsnummer DSM 13182, hinterlegt am 06.12.1999 bei der Deutschen Sammlung von Mikroorganismen und Zellkulturen in Braunschweig, eingesetzt.

Selbstverständlich können die eingesetzten Bakterien natürlich vorkommende oder genmanipulierte Bakterien, insbesondere Bacillen sein. Beispielsweise können die eingesetzten Bakterien Resistenzen gegen bestimmte Antibiotica aufweisen, um so eine besonders einfache Produktion des enzymatisch wirkenden Agens zu ermöglichen. Das aus den Bakterien stammende Enzym kann natürlichen Ursprungs sein beziehungsweise eine Wildtyp-Amino-säuresequenz aufweisen, es kann aber auch Aminosäureabweichungen gegenüber dem natürlicherweise vorkommenden Enzym aufweisen, beispielsweise Aminosäuredeletionen, -insertionen, -inversionen, -austau-sche oder -additionen, auch ungewöhnlicher Aminosäuren. Das Enzym kann gegebenenfalls auch Modifikationen, wie Glycosylierungen oder ähnliches, aufweisen. Das Enzym kann auch als Fusionsprotein mit einem anderen Protein oder Peptid oder als Enzymfragment vorliegen, solange es in der Lage ist, Galactomannane zu den vorgenannten Produkten zu hydrolysieren.

Die Erfindung betrifft auch die Verwendung des enzymatisch wirkenden Agens zur Hydrolyse des Galactomannans, wobei das enzymatisch wirkende Agens in freier oder in immobilisierter Form für die Hydrolyse eingesetzt werden kann. So kann erfindungsgemäß die Hydrolyse mit ruhenden Zellen von Bakterien, vorzugsweise Bacillus subtilus, durchgeführt werden. Es kann auch vorgesehen sein, die Zellen zuerst in einer stabilen inerten Matrix zu immobilisieren und den so entstandenen Biokatalysator für die Hydrolyse des Galactomannans einzusetzen. Erfindungsgemäß können sowohl die Enzyme, die Bakterien oder auch der Rohextrakt immobilisiert werden. Die Immobilisierung kann durch Bindung an Träger, Quervernetzung, Einschluss oder Einkapselung erfolgen. Die Quervernetzung kann beispielsweise durch Glutaraldehyd erfolgen. Die Trägerbindung kann durch Adsorptionsbindung oder kovalente Bindung erfolgen, während für den Einschluss zum Beispiel semipermeable Membrane in Form von Gelen, Mikrokapseln oder Fasern eingesetzt werden. Eingekapselte Enzyme oder Mikroorganismen sind durch eine semipermeable Membran von der umgebenden Substrat- und Produktlösung getrennt.

Die Erfindung betrifft auch ein vorgenanntes Verfahren, wobei das erhaltene Gemisch aus Mannoseund Galactose-haltigen Oligosacchariden einem chromatographischen Trennverfahren unterzogen wird, bei dem erwünschte Oligosaccharide mit bestimmtem Polymerisationsgrad erhalten werden können.

Weitere vorteilhafte Ausgestaltungen der Erfindung ergeben sich aus den Unteransprüchen.

Die Erfindung wird anhand der folgenden Beispiele näher erläutert:

### Beispiel 1: Herstellung des Biokatalysators

Eine Abimpfung des Stammes Bacillus subtilis SZ 100 (DSM 13182) wird von einer Schrägagar-Kultur in einen Schüttelkolben mit einem Medium bestehend aus Caseinpepton (15/g/L), Sojamehlpepton (5/g/L), NaCl (5/g/L) und Guargum (1/g/L) eingebracht und 24 Stunden unter Schütteln bei 30°C inkubiert.

Danach wird die Schüttelkultur in einen 10 L-Fermenter transferiert und in einem Medium gleicher Zusammensetzung wie das der Schüttelkultur weiterkultiviert.

Nach 24 Stunden Wachstum werden die Zellen abzentrifugiert, in einer 3 %igen Natriumalginatlösung resuspendiert und dann unter Rühren in eine 2 %ige Calciumchloridlösung eingetropft. Die gebildeten, mit Bacillus subtilis (DSM 13182) Zellen beladenen Calciumalginatkugeln (Biokatalysator) werden gewaschen, getrocknet und kühl gelagert.

### Beispiel 2: Hydrolyse des Guargums

Der nach Beispiel 1 hergestellte Biokatalysator wird in einer wässrigen Guargum-Lösung (Konzentration 1 bis 5 %) aufgequollen und in eine auf 37°C temperierte Säule gefüllt. Die Hydrolyse des Guargums erfolgt dann kontinuierlich durch Durchleiten einer Guargum-Lösung durch die Säule. Der Durchlauf wird auf seinen Gehalt an Mono-, Oligound Polysacchariden per HPLC analytisch untersucht und folgende Zusammensetzung erhalten:

| | |
|---|---|
| Monosaccharide | 2 % |
| Oligosaccharide | 70 % |
| Polysaccharide | 28 %. |

Die Hydrolyse kann auch semikontinuierlich oder diskontinuierlich durchgeführt werden. Für letzteres wird der nach Beispiel 1 hergestellte Biokatalysator in einem Rührreaktor mit einer Guargum-Lösung in Kontakt gebracht.

Es ist auch möglich, für die Hydrolyse einen Enzym-Rohextrakt aus Bacillus subtilis (DSM 13182) einzusetzen. Hierzu wird die Biomasse nach Fermentation durch Zentrifugation isoliert, in Phosphat-Puffer resuspendiert und anschließend aufgeschlossen (Ultraschall, French Press, Kugelmühle, etc.). Die Zelltrümmer werden durch Zentrifugation entfernt und der so erhaltene Rohextrakt ohne weitere Aufreinigung der zu hydrolysierenden Guargum-Lösung zugesetzt.

Die nach der jeweiligen Hydrolyse erhaltene wässrige Lösung enthält neben den gewünschten Galacto-Manno-Oligosacchariden mit DP < 15, insbesondere DP 2 bis 7, auch einen geringen Anteil höhermolekularer Bestandteile. Diese können sehr leicht durch an sich bekannte Trennverfahren, zum Beispiel Chromatographie an calciumbeladenen starksauren Kationenaustauschern oder fraktionierte alkoholische Fällung oder Ultrafiltration entfernt werden, so dass man die Galacto-Manno-Oligosaccharide DP < 15, insbesondere DP 2 bis 7, in reiner Form in einer wässrigen Lösung erhält, aus der sie mit an sich bekannten Methoden (zum Beispiel durch Sprühtrocknung) trocken gewonnen werden können.

### Beispiel 3: Stabilität der Galacto-Manno-Oligosaccharide im Mund, Magen und Dünndarm

### Stabilität im Mundbereich:

Die Stabilität der gemäß Beispiel 2 erhaltenen erfindungsgemäßen Oligosaccharide gegenüber der Mundflora wurde mit den im Mundbereich vorkommenden Bakterienstämmen Streptococcus mutans DSM 20523 und Streptococcus sobrinus NCTC 10922 sowie mit frischem Zahnplaque untersucht.

Streptococcus mutans und Streptococcus sobrinus wurden in flüssigem DSM-Medium 92 unter anaeroben Bedingungen 24 Stunden bei 37°C kultiviert. Am Ende der logarithmischen Wachstumsphase wurden die Zellen abzentrifugiert (15 min., 4000 x g) und in 10 % des ursprünglichen 20 mM Carbonatpuffer, pH 7,5 resuspendiert. Anschließend wurden 9 mL einer Lösung der erfindungsgemäßen Oligosaccharide (1 %ig in 20 mM Carbonatpuffer, pH 7,5) mit 1 mL Bakterien-Suspension beimpft und 2 Stunden bei 37°C inkubiert. Zu verschiedenen Zeitpunkten wurden Proben entnommen und auf ihren Oligosaccharidgehalt untersucht (Ergebnisse siehe unten):

Drei freiwilligen männlichen Personen, die drei Tage ihre Zähne nicht gereinigt hatten, wurden Plaqueproben entnommen und jeweils 10 mg Plaque in 1 mL Oligosaccharidlösung (1 %ig in 20 mM Carbonatpuffer, pH 7,5) suspendiert. Während der zweistündigen Inkubationszeit bei 37°C wurden Verlaufsproben entnommen und auf ihren Oligosaccharidgehalt untersucht.

### Ergebnis:

Während die als Kontrolle mitgeführte Saccharose innerhalb von 120 Minuten vollständig sowohl durch die beiden Streptococcen-Stämme als auch durch die Mischkultur des Plaque hydrolysiert wurde, war eine Spaltung der erfindungsgemäßen Oligosaccharide auch nach 2 Stunden nicht zu erkennen.

### Stabilität im Magen:

Die Stabilität einer Substanz bei der Magen-Passage kann durch die Bestimmung der Hydrolyserate bei pH 1,0 beziehungsweise 2,0 dargestellt und mit Saccharose als Kontrolle verglichen werden:

Hierzu wurden 1 %ige Galacto-Manno-Oligosaccharidlösungen bei pH-Wert 1,0 (0,1 M HCl) und pH-Wert 2,0 (0,01 M HCl) bei 37°C für 3 Stunden inkubiert. Aus dem Reaktionsansatz wurden nach 60, 120 und 180 Minuten Proben entnommen und diese mittels HPAEC analysiert. Dabei wurden Saccharose und 1-Kestose als Kontrollsubstanzen verwendet.

**Tabelle I**

| Ergebnisse: Angabe als Hydrolyserate in %: | | | | |
|---|---|---|---|---|
| Substanz | pH | Inkubationszeit [Min] | | |
| | | 60 | 120 | 180 |
| Saccharose | 1 | 24 | 55 | 61 |
| | 2 | 1 | 2 | 7 |
| 1-Kestose | 1 | 90 | 100 | 100 |
| | 2 | 16 | 30 | 43 |
| Galacto-Manno-Oligos | 1 | < 1 | < 1 | < 1 |
| | 2 | < 1 | < 1 | < 1 |

Aus der Tabelle I ist ersichtlich, dass die Galacto-Manno-Oligosaccharide eine Magen-Passage unbeschadet überstehen können.

### Stabilität gegenüber Pankreas-Enzymen:

Das Pankreas-Sekret enthält eine große Anzahl von Hydrolasen, unter anderem auch kohlenhydratspaltende Enzyme wie α-Amylase, welche α-1,4-Glucane (Stärke, Glykogen) bevorzugt zu Maltose und Maltooligosacchariden spaltet.

Die Prüfung der Stabilität von Galacto-Manno-Oligosacchariden gegenüber Pankreas-Enzymen wurde wie folgt durchgeführt:

### Benötigte Lösungen:

• 20 mM Na-Phosphat-Puffer, pH 7,0 plus 6 mM NaCl (Lösung 1)
• 1 %ige Stärkelösung (lösliche Stärke nach Zulkowski) in Lösung 1
• 1 %ige Galacto-Manno-Oligosaccharidlösung in Lösung 1
• 0,2 % Pankreatin (Fa. Sigma) gelöst in Lösung 1

**Tabelle II**

| Reaktionsansätze: | | |
|---|---|---|
| Komponenten | Probe | Kontrolle |
| Galacto-Manno-Oligosaccharid-Lösung | 3,0 ml | - |
| Stärke-Lösung | - | 3,0 ml |
| Enzymlösung | 0,1 ml | 0,1 ml |

Nach 210 Minuten Inkubation im Thermomixer (Intervall-Schütteln) bei 37°C wurde die Reaktion durch 15 min. Erhitzen auf 95°C beendet und die Proben mittels HPAEC analysiert. Dabei wurde die stärkehaltige Probe zuvor durch 3-stündiges Erhitzen in 1 M HCl bei 95°C vollständig hydrolysiert.

**Tabelle III**

| Ergebnisse: | |
|---|---|
| Substanz | Abbaurate (%) |
| Stärke | 77 |
| Galacto-Manno-Oligosaccharide | 0 |

Die Tabelle III zeigt, dass die erfindungsgemäßen Galacto-Manno-Oligosaccharide durch die Pankreas-Enzyme nicht angegriffen werden.

### Beispiel 4: Spaltbarkeit durch Dünndarm-α-Glucosidasen

Die im Dünndarm vorhandenen mucosaständigen Enzym-Komplexe Saccharase/Isomaltase und Glucoamylase/Maltase sorgen in vivo dafür, dass die in den Dünndarm gelangten Disaccharide Maltose und Saccharose und zum Teil auch Maltooligosaccharide zu Monosacchariden gespalten werden und als solche über die Darmwand in den Blutkreislauf gelangen können.

Die Prüfung der Stabilität von erfindungsgemäßen Galacto-Manno-Oligosacchariden gegenüber diesen Enzymen wurde wie folgt durchgeführt:

### Enzym-Isolierung:

Die Enzym-Komplexe Saccharase/Isomaltase (SI-Komplex) und Glucoamylase/Maltase (GM-Komplex) wurden aus Schweine-Dünndarm nach der Methode von H. Heymann (Dissertation, Hannover, 1991) isoliert.

Die Spaltbarkeit der erfindungsgemäßen Galacto-Manno-Oligosaccharide durch Dünndarm-α-Glucosidasen wurde wie folgt bestimmt:

### Benötigte Lösungen:

- Triethanolamin (TRA)-Puffer, 0,1 M, pH 7,0
- Galacto-Manno-Oligosaccharide, 1 %ige Lösung in TRA-Puffer
- Maltose, beziehungsweise Saccharose als Kontrollsubstanzen, 1 %ig in TRA-Puffer
- Mucosa-Enzym, gelöst in TRA-Puffer

### Reaktionsansatz:

Zu 1,2 mL der auf 37°C temperierten Kohlenhydratlösung wurden 0,7 U des Enzymkomplexes Saccharase/Isomaltase beziehungsweise Glucoamylase/Maltase zu t = O zugegeben, gemischt und bei 37°C inkubiert. Die Reaktion wurde nach 2 Stunden durch 15-minütiges Erhitzen auf 95°C gestoppt. Die gebildeten Monosaccharide sowie die eingesetzten Testsubstanzen wurden mittels HPAEC quantitativ bestimmt.

**Tabelle IV**

| Ergebnisse: | | |
|---|---|---|
| Kohlenhydrat | Enzymkomplex | Hydrolyserate (%) |
| Saccharose | SI | 98 |
| Maltose | SI | 95 |
| Maltose | GM | 96 |
| Galacto-Manno-Oligos. | SI | < 1 |
| Galacto-Manno-Oligos. | GM | < 1 |

Die Ergebnisse zeigen, dass unter den gewählten Bedingungen einer fast vollständigen Hydrolyse von Saccharose beziehungsweise Maltose im Falle des SI-Enzymkomplexes sowie von Maltose im Falle des GM-Enzymkomplexes die Galacto-Manno-Oligosaccharide durch beide Enzymkomplexe praktisch nicht gespalten werden.

### Beispiel 5: Spaltbarkeit durch isolierte Enzym-Komplexe (SI-Komplex und GM-Komplex)

Die isolierten Enzym-Komplexe Saccharase/Isomaltase (SI-Komplex) und Glucoamylase/Maltase (GM-Komplex) aus Schweine-Dünndarm (siehe Beispiel 4) sind für Inhibitionsuntersuchungen mit erfindungsmäßigen Galacto-Manno-Oligosacchariden in Gegenwart der Substrate Saccharose beziehungsweise Maltose im Falle des SI-Komplex und Maltose im Falle des GM-Komplex getestet worden. Das Substrat-Inhibitor-Verhältnis betrug in allen Fällen 10:1.

| | |
|---|---|
| Ansatz | - 0,7 ml Substratlösung, 1,43 % in 0,1 M Na-Phosphat-Puffer, pH 7,0 - 0,1 ml Galacto-Manno-Oligosaccharide, 1 % - 0,1 ml 0,1 M Na-Phosphat-Puffer, pH 7,0 |
| | |
| Vorinkubation | 15 min, 37°C Entnahme der Nullprobe |
| | |
| Start | 0,1 ml Enzymlösung (0,5 U/ml Maltase-Aktivität im Ansatz) |
| | |
| Probenahme | je 0,15 ml Probe nach 30 und 60 min |
| | |
| Reaktionsstop | 2 min bei 95°C |
| | |
| Analytik | HPAEC, Standard-Lösung je 10 ppm Glucose, Fructose, je 20 ppm Saccharose, Maltose |

**Tabelle V**

| Ergebnis: | | |
|---|---|---|
| **% Inhibierung** | **Inkubationsdauer** | |
| | 30 Min. | 60 Min. |
| SI/Saccharose | 24 | 25 |
| SI/Maltose | 26 | 18 |
| GM/Maltose | 0 | 0 |

Wie der Tabelle V zu entnehmen ist, wird die Spaltung der Saccharose beziehungsweise Maltose durch den SI-Enzym-Komplex in Gegenwart von Galacto-Manno-Oligosacchariden inhibiert. Die Maltose-Spaltung durch den GM-Komplex wird durch Zugabe von Galacto-Manno-Oligosacchariden hingegen nicht beeinflusst.

### Beispiel 6: Verhinderung der Anlagerung von pathogenen Keimen an Epithelzellen

### Epithelzellen

Menschliche Uroepithelzellen, gewonnen durch Zentrifugation aus dem Morgenharn

### Keime

Staphylococcus aureus, 2 Stämme und E. coli, 2 Stämme, jeweils als Suspension mit 10⁹ Keimen/mL

### Testdurchführung

Epithelzellen und Keimsuspension wurden zusammengebracht und bei 37°C für 30 Minuten inkubiert. Dann wurden die Epithelzellen von den nicht adhärenten Keimen durch Membranfiltration (8µ) abgetrennt. Die Filter wurden mehrfach gewaschen, in physiologische Kochsalzlösung eingebracht und die Epithelzellen darin suspendiert. Nach Zentrifugation der Suspension in Kochsalzlösung wurde das Pellet auf Objektträger aufgetragen, und nach May-Grünwald und Giemsa gefärbt. Die Anzahl der an 50 Epithelzellen anhaftenden Keime wurde gezählt. Die Zahl ergab den Leerwert. Als Kontrolle dienten Epithelzellen ohne Zugabe einer Keimsuspension.

In dem Hauptversuch wurden Epithelzellen zunächst mit unterschiedlich konzentrierten Galacto-Manno-Oligosaccharidlösungen für 1, 2 beziehungsweise 3 Stunden inkubiert. Dann wurden sie mit der Keimsuspension zusammengebracht und -wie oben beschriebenweiterbehandelt. Die Zählung der anhaftenden Keime an 50 Epithelzellen ergab den Messwert.

### Ergebnis:

Bei den als Vergleich eingesetzten "neutralen" Kohlenhydraten, wie zum Beispiel Raffinose, Nystose und Isomelezitose, wurde keine Verminderung der Keimanlagerung an den Epithelzellen festgestellt. Mit den erfindungsgemäßen Galacto-Manno-Oligosacchariden wurde die Adhäsion aller geprüften Mikroorganismen fast vollständig (Blockierung: > 95 %) verhindert.

### Beispiel 7: Erhöhung der Mucussekretion im Dickdarm

Aus resektiertem Dickdarm von frisch geschlachtetem Schwein wurden nach gründlichem Säubern 1 cm² große Stücke aus dem distalen Abschnitt ausgestanzt. Die so präparierten Stücke wurden in Hanks-Puffer, dem Chloramphenicol und Ampicillin (je 50 µg/mL) zugesetzt worden waren, unter Sauerstoff-Begasung und Rühren bei 37°C für 5 Stunden inkubiert. Der Puffer enthielt außerdem 1 % der auf ihre stimulierende Wirkung auf Mucussekretion zu prüfenden, erfindungsgemäß hergestellten Galacto-Manno-Oligosaccharide. Es wurden jeweils 10 Darmstücke zur Kontrolle beziehungsweise in dem Testansatz eingesetzt. Einer der Ansätze enthielt das zur Therapie von entzündlichen Darmerkrankungen üblicherweise verwendete Hydrocortison.

Als Maß für eine Erhöhung der Mucussekretion wurde die Zunahme des Gesamtkohlenhydratgehalts im Überstand gemessen. Hierzu wurden während der Inkubation Proben zu verschiedenen Zeitpunkten entnommen und zu jeweils 10 µL der Probe 20 µL Resorcinlösung (6 mg/mL) und 100 µL 75-%ige Schwefelsäure zugegeben und nach 60 min Inkubation bei 80°C die Extinktion bei λ = 450 nm gemessen.

### Ergebnis:

Die erfindungsgemäßen Galacto-Manno-Oligosaccharide erhöhen die Mucussekretion um das circa 2,4-fache gegenüber der Kontrolle, während die Erhöhung durch das Hydrocortison das circa 5,3-fache beträgt. In neueren Untersuchungen wurde für Substanzen wie Corticosteroide gezeigt, dass sie in Zellkulturen, die aus Colonepithel-Biopsien stammten, eine Stimulation der endogenen Mucussekretion bewirken (Finnie I.A., et al. Clinical Science, 91 (1996), 359-364). Somit ergibt sich die Möglichkeit, entzündliche Darmerkrankungen durch Nahrungsmittelbestandteile, die aus erfindungsgemäßen Galacto-Manno-Oligosacchariden bestehen, günstig zu beeinflussen.

### Beispiel 8: Einfluss auf die Mikroflora im Dickdarm

Um den Einfluss der erfindungsgemäßen Galacto-Manno-Oligosaccharide auf die Zusammensetzung der Mikroflora im Dickdarm zu untersuchen, wurden verschiedene Reinkulturen von in Humanfaeces vorkommenden Bakterien im folgenden Medium mit Galacto-Manno-Oligosacchariden als einziger Kohlenstoffquelle kultiviert:

| | |
|---|---|
| Trypticase/Trypton | 1,50 g |
| Hefe-Extrakt | 1,00 g |
| KH₂PO₄ | 0,24 g |
| Na₂HPO₄ | 0,24 g |
| (NH₄)₂SO₄ | 1,24 g |
| NaCl | 0,48 g |
| MgSO₄.7H₂O | 0,10 g |
| CaCl₂.2H₂O | 0,06 g |
| FeSO₄.7H₂O | 2 mg |
| Resazurin | 1 mg |
| Cystein/HCI | 0,50 g |
| Vitaminlösung (nach DSM 141) | 0,50 mL |
| Spurenelementelösung (nach DSM 141) | 9,00 mL |
| NaHCO₃ | 2,00 g |
| Galacto-Manno-Oligosaccharide | 5,00 g |
| H₂O dest. ad 1000 mL, pH 7,0 | |

Der Test wurde mit folgenden Mikroorganismen durchgeführt:
Bacteroides asaccharolyticus
Bacteroides distasonis
Bacteroides fragilis
Bacteroides tetaiotaomicron
Bifidobacterium adolescentis
Bifidobacterium bifidum
Bifidobacterium breve
Bifidobacterium infantis
Bifidobacterium longum
Eubacterium lentum
Eubacterium limosum
Lactobacillus casei
Lactobacillus fermentum

Clostridium butyricum
Clostridium difficile
Clostridium perfringens
Enterobacter cloacae
Escherichia coli
Klebsiella pneumoniae
Salmonellla typhimurium
Serratia marcescens
Staphylococcus aureus

Die Mikroorganismen wurden jeweils 24 Stunden bei 37°C unter anaeroben Bedingungen kultiviert. Während der Versuchsdauer wurden Proben zu bestimmten Zeitpunkten entnommen und auf pH-Wert und Oligosaccharid-Konzentration untersucht. Außerdem wurde mit Hilfe der optischen Dichte die Zunahme der Zellzahl bestimmt.

Lediglich die Bifidobacterien und die beiden Lactobacillen waren in der Lage, die erfindungsgemäßen Galacto-Manno-Oligosaccharide zu verstoffwechseln und darauf zu wachsen. Bei allen anderen untersuchten Bakterien konnte kein Wachstum auf dem Medium festgestellt werden. Somit ergibt sich, dass Nahrungsmittel, die erfindungsgemäße Galacto-Manno-Oligosaccharide enthalten, die Zusammensetzung der Dickdarm-Mikroflora positiv verändern können.

### Beispiel 9: Stärkung der Immunabwehr

### Stärkung der Phagocytose

Der Einfluss von erfindungsgemäßen Galacto-Manno-Oligosacchariden auf die Funktion der Phagocyten (Mono-, Granulocyten) lässt sich mittels Phagocytoseassays ermitteln. Bei der Phagocytose-Stimulation wurden die Phagocyten mit Galacto-Manno-Oligosacchariden vorab inkubiert (100 µg Glykan/0,11 ml Vollblut, 37°C, 10 Min.). Ein korrespondierender Leerwert wurde entsprechend 10 Minuten im Eisbad inkubiert. Danach erfolgte der eigentliche Phagocytoseassay unter folgenden Bedingungen:

0,11 ml Vorinkubationslösung wurde für 10 Minuten im Eisbad belassen, dann wurden 0,01 ml nicht opsonierte E. coli (Fluoresceinisothiocyanat (FITC)-markiert, 10⁹ pro ml, ORPEGEN) oder 0,01 ml nicht opsonierte Staphylococcus aureus (15 x 10⁶ pro ml, FITC-markiert, MOLECULAR PROBES) hinzugefügt. Die Ansätze wurden (Doppelbestimmungen) bei 37°C 10 Minuten inkubiert. Die Ansätze wurden zweimal mit je 3 mL Waschpuffer (ORPEGEN) gewaschen und bei je 250 xg für 5 Minuten bei 4°C zentrifugiert.

Die Lyse der Erythrocyten im Sediment erfolgte mit 2 ml Lysepuffer (ORPEGEN) 20 Minuten bei Raumtemperatur und anschließend wurde der Ansatz einmal gewaschen. Zum Sediment wurde 0,2 ml DNA-Färbelösung (Propidiumjodid) gegeben und für 10 Minuten im Eisbad belassen.

Die Messung erfolgte im Durchflusscytometer (Anregung: 488 nm, Emission FL-1 : 520 nm), wobei zwischen Monocyten und Granulycyten ohne Separation unterschieden werden kann (Vorwärtsstreulicht (FSC) versus (ESC) Seitwärtsstreulicht). Die Resultate sind ausgedrückt als %-Anteil der Phagocytosepositiven Zellen (siehe folgende Tabelle VI):

Als Kontrollsubstanz ohne Phagocytosewirkung wurde Raffinose eingesetzt.

**Tabelle VI**

| **Phagocytoseassay** | | | |
|---|---|---|---|
| **Einfluss von Galacto-Manno-Oligosacchariden (100 µg/Ansatz)** | | | |
| Zelltyp | Monocyten (% positive Zellen) | Granulocyten (% positive Zellen) | |
| **Bakterienart** | **E. coli** | **E. coli** | **Staph. aureus** |
| | nicht opsoniert | nicht opsoniert | nicht opsoniert |
| Kontrolle 4°C | 8,0 | 1,0 | 0,5 |
| Kontrolle 37°C | 21,5 | 19,5 | 27,0 |
| Galacto-Manno-Oligos. | 35,0 | 33,5 | 42,5 |
| Raffinose | 22,0 | 17,0 | 30,0 |

Die Ergebnisse zeigen, dass nach Vorinkubation der Mono- beziehungsweise Granulocyten mit den erfindungsgemäßen Galacto-Manno-Oligosacchariden eine Stimulation der Phagocytose erreicht wurde.

### Adhäsionsassay

Im Adhäsionsassay wurde der Einfluss von erfindungsgemäßen Galacto-Manno-Oligosacchariden auf das Adhäsionsverhalten von B-Zell-Linien (zum Beispiel Reh) und normalen humanen B-Lymphocyten an epithelialen Zellen (Colontumor-Linie HT 29) gemessen. Bei dem verwendeten Overlay-Assay wurden die Lymphocyten intrazellulär mit Calcein markiert, um die Messung fluorometrisch durchführen zu können.

Zunächst erfolgte eine Beschichtung mit der adhärenten Colonepithelzell-Linie (HT 29) in Mikrotiterplatten (24 Loch-Costar®-Platten). Man inkubiert (37°C, über Nacht) bis sich ein konfluenter Rasen ausgebildet hat. Dies erfolgte soweit möglich im Serum-freien Medium. Einsaat: 1 x 10⁴ Zellen. Die adhärenten Zellen wurden dann dreimal mit PBS (phosphatgepufferte physiologische NaCl-Lösung) vor dem nachfolgenden Assay gewaschen und entsprechend mit PBS + 1 % Rinderserum-Albumin (BSA) 1 Stunde bei Raumtemperatur blockiert.

Die zu testenden B-Zell-Linien oder isolierten B-Lymphocyten wurden unbehandelt mit Calcein fluoreszent markiert (1 x 10⁷ Zellen in 2 ml RPMI-Medium + HEPES + 10 µl Calcein AM, 30 Minuten, 37°C; danach 2 x mit HBSS (Hanks-Puffer) + 0,25 % BSA gewaschen) und 1 x 10⁶ Zellen in 0,5 ml PBS + 0,25 % HBSS pro Loch 1 Stunde inkubiert. Die Kulturplatte wurde 10 Sekunden auf den Schüttler gestellt, pro Loch 0,5 ml 0,2 % Glutaraldehyd in PBS + 0,2 % BSA dazugegeben und 10 Minuten auf dem Schüttler inkubiert. Die Fluoreszenz der Platte wurde in einer Fluoreszenzmessvorrichtung gemessen (494, 517 nm).

Die erste Messung entspricht dem 100-%-Wert. Danach wurde die Platte vier mal gewaschen und erneut gemessen. Diese Messung entspricht der spezifischen Adhäsion, welche prozentual zum 100-%-Wert abzüglich des Autofluoreszenz-Wertes (Zellen unmarkiert) angegeben wird. Als Kontrollsubstanzen wurden Galactose beziehungsweise Glucose eingesetzt.

Adhäsion von Reh (prä-B) auf der Colon-Zellinie HT 29

**Tabelle VII**

| Einfluss von Galacto-Manno-Oligosacchariden (10 µg/Ansatz) | | | |
|---|---|---|---|
| **Zucker** | **Mittelwert (%)** | **SD** | **Index** |
| Ohne Kohlenhydrat | 46 | 6,3 | 1,00 |
| D-Glucose | 47 | 6,0 | 1,11 |
| D-Galactose | 51 | 7,0 | 1,12 |
| Galacto-Manno-Oligosaccharide | 93 | 9,7 | 2,02 |

Gegenüber den Kontrollsubstanzen D-Glucose und D-Galactose ergab sich in Gegenwart der Galacto-Manno-Oligosaccharide ein deutlich verstärktes Adhäsionsverhalten der B-Zell-Linie (Reh) auf der Colonzell-Linie HT 29.

### Beispiel 10: Verbesserung der Aufnahme von Calcium

### Versuchsvorbereitung:

Männliche Sprague-Dawley Ratten mit je circa 100 g Gewicht wurden mit freiem Zugang zu entmineralisiertem Wasser und folgender Standarddiät für eine Gewöhnungsphase von 4 Tagen gehalten:

| | |
|---|---|
| Casein | 250 g |
| Maiskeimöl | 50 g |
| Mineralsalzmischung (Ca- und Fe-frei) | 25 g |
| Calciumcarbonat | 7,5 g |
| Vitamin-Mischung | 10 g |
| Vitamin E | 1 g |
| Cholinbitartarat | 4 g |
| Saccharose | ad 1000 g |

Danach wurden sie in zwei Gruppen unterteilt. In der ersten Gruppe wurde den Tieren der Magen vollständig entfernt (Magen-Resektion-Gruppe), die zweite Gruppe diente als Kontrolle (KontrollGruppe). Nach der Operation wurden die Ratten 24 Stunden ohne Nahrung und Wasser, danach 2 bis 3 Tage an Kuhmilch und dann 14 bis 16 Tage mit Standarddiät für eine Gewöhnungsphase von 4 Tagen gehalten:

### Versuchsdurchführung

Die Testgruppen wurden wiederum in jeweils zwei Untergruppen unterteilt. Jeweils eine der Untergruppen wurde weiter mit Standarddiät gehalten, während die andere Untergruppe eine Diät mit Galacto-Manno-Oligosaccharid-Zusatz (50 g/kg Diät) erhielt. Über eine Versuchsdauer von 3 Wochen wurden ab dem dritten Tag Faeceproben gesammelt und auf ausgeschiedenes Calcium untersucht. Am Ende des Versuchs wurde der Inhalt des Caecums analysiert.

### Ergebnisse:

Mit Standarddiät war die Calciumaufnahme der Magen-Resektion-Gruppe nur etwa ein Viertel der Calciumaufnahme der Kontrollgruppe.

Durch die Beimischung von erfindungsgemäßen Galacto-Manno-Oligosacchariden konnte die Calciumaufnahme der Magen-Resektion-Gruppe etwa verdoppelt werden, so dass sie auf etwa 50 % der der KontrollGruppe anstieg.

In den an der erfindungsgemäßen Galacto-Manno-Oligosaccharid-haltigen Diät gehaltenen Ratten zeigte die Analyse des Caecuminhaltes eine stark erhöhte Propionsäurekonzentration. Diese steht in signifikantem Zusammenhang mit der gemessenen Calciumaufnahme.

## Patentansprüche

1. Verfahren zur Herstellung von Mannose- und Galactose-haltigen Oligosacchariden aus Galactomannanen, wobei eine wässrige Lösung oder Suspension des Galactomannans hergestellt, unter Einsatz eines enzymatisch wirkenden Agens aus Bakterien des bei der DSM unter Nr. 13182 hinterlegten Stamms der Art *Bacillus subtilis* hydrolysiert und eine wässrige Lösung eines Gemischs aus Galacto-Manno-Oligosacchariden mit einem Polymerisationsgrad (DP) < 15 erhalten wird.

2. Verfahren nach Anspruch 1, wobei der Polymerisationsgrad (DP) 2 bis 7 beträgt.

3. Verfahren nach Anspruch 1 oder 2, wobei das enzymatisch wirkende Agens lebende oder tote Zellen von Bakterien sind.

4. Verfahren nach Anspruch 1 oder 2, wobei das enzymatisch wirkende Agens ein Galactomannanhydrolysierendes Enzym oder ein Rohextrakt aus den Zellen dieser Bakterien ist.

5. Verfahren nach einem der Ansprüche 1 bis 4, wobei die enzymatische Hydrolyse mit immobilisierten Zellen, immobilisierten Rohextrakten oder immobilisierten Enzymen aus diesen Bakterien durchgeführt wird.

6. Verfahren nach einem der vorhergehenden Ansprüche, wobei das erhaltende Gemisch aus Galacto-Manno-Oligosacchariden einem chromatographischen Trennverfahren unterzogen wird.

7. Galacto-Manno-Oligosaccharid, herstellbar aus Guar-Gum nach einem Verfahren der Ansprüche 1 bis 6, umfassend β-1,4-verknüpfte Mannoseeinheiten und daran α-1,6-verknüpfte Galartosesinheiten mit einem Polymerisationsgrad (DP) < 15, insbesondere 2 bis 7.

8. Galacto-Manno-Oligosaccharid, herstellbar aus Guar-Gum nach einem Verfahren der Ansprüche 1 bis 6, umfassend β-1,4-verknüpfte Mannoseeinheiten und daran α-1,6-verknüpfte Galactoseeinheiten mit einem Polymerisationsgrad < 15, insbesondere 2 bis 7, zur Verwendung als therapeutisches oder diagnostisches Mittel.

9. Enzym mit der Fähigkeit, verschiedenartige Galactomannane, bevorzugt Guar-Gum, Cassia-Gum und Jonannisbrot-Kernmehl, mit jeweils annähernd gleicher Effizienz zu hydrolysieren, herstellbar aus dem Bacillus subtilis-Stamm DSM 13182.

10. Rohextrakt mit der Fähigkeit, verschiedenartige Galactomannane, bevorzugt Guar-Gum, Cassia-Gum und Jonannisbrot-Kernmehl, mit jeweils annähernd gleicher Effizienz zu hydrolysieren, herstellbar durch Zellaufschluss von Bakterien des Stammes Bacillus subtilis DSM 13182.

11. Bacillus subtilis-Stamm DSM 13182.

12. Arzneimittel, enthaltend ein Galacto-Manno-Oligosaccharid nach Anspruch 7, gegebenenfalls zusammen mit einem pharmazeutisch verträglichen Träger.

13. Lebens- oder Genussmittel, enthaltend ein Galacto-Manno-Oligosaccharid nach Anspruch 7.

14. Verwendung eines Galacto-Manno-Oligosaccharids nach Anspruch 7 zur Herstellung von Genussund/oder Lebensmitteln und/oder zur Senkung des glykämischen Index von Genuss- und/oder Lebensmitteln.

15. Verwendung eines Galacto-Manno-Oligosaccharids nach Anspruch 7 zur Herstellung eines dieses Galacto-Manno-Oligosaccharid enthaltenden Lebensmittels zur Verhinderung von Infektionskrankheiten, zur Verhinderung von Darmerkrankungen, zur Verhinderung der Coloncarzinogenese, zur Stärkung der Immunabwehr gegenüber allgemeinen Infekten, zur Verhinderung von entzündlichen Erkrankungen und/oder zur Verhinderung der Osteoporose.

16. Verwendung eines Galacto-Manno-Oligoaaccharids nach Anspruch 7 zur Herstellung eines Arzneimittels zur Verhinderung von Infektionskrankheiten, zur Verhinderung von Darmerkrankungen, zur Verhinderung der Coloncarzinogenese, zur Stärkung der Immunabwehr gegenüber allgemeinen Infekten, zur Verhinderung von entzündlichen Erkrankungen und/oder zur Verhinderung der Osteoporose.

## Claims

1. Method for producing oligosaccharides containing mannose and galactose from galactomannans, according to which an aqueous solution or suspension of the galactomannan is produced, which is hydrolysed by means of an enzymatically effective agent obtained from bacteria of the *Bacillus subtilis* type of the strain deposited with DSM under no. 13182, and an aqueous solution of a mixture of oligosaccharides containing mannose and galactose with a degree of polymerisation (DP) of <15 is obtained.

2. Method according to claim 1, wherein the degree of polymerisation (DP) is 2 to 7.

3. Method according to claim 1 or 2, wherein the enzymatically active agent constitutes live or dead cells of bacteria.

4. Method according to claim 1 or 2, wherein the enzymatically effective agent is an enzyme which hydrolyses galactomannans from bacteria or is a raw extract from cells of said bacteria.

5. Method according to one of the claims 1 to 4, wherein the enzymatic hydrolysis is carried out with immobilised cells, immobilised raw extracts or immobilised enzymes of said bacteria.

6. The method according to one of the preceding claims, wherein the mixture of oligosaccharides containing galactose and mannose obtained is subjected to a chromatographic separating process.

7. A galactomanno-oligosaccharide which can be produced from guar gum according to one of the methods described in claims 1 to 6, comprising β-1,4-linked mannose units linked to α-1,6-linked galactose units with a degree of polymerisation (DP) of <15, in particular of 2 to 7.

8. A galactomanno-oligosaccharide which can be produced from guar gum according to one of the methods described in claims 1 to 6, comprising β-1,4-linked mannose units linked to α-1,6-linked galactose units with a degree of polymerisation of <15, in particular of 2 to 7, for use as a therapeutic or diagnostic agent.

9. An enzyme with the ability to hydrolyse various types of galactomannans, preferably guar gum, cassia gum and locust bean gum, with an approximately equal efficiency in each case, said enzyme being able to be produced from the *Bacillus subtilis* strain DSM 13182.

10. A raw extract with the ability to hydrolyse various types of galactomannans, preferably guar gum, cassia gum and locust bean gum, with an approximately equal efficiency in each case, said raw extract being able to be produced by means of a disruption of cells of bacteria of the *Bacillus subtilis* strain DSM 13182.

11. The *Bacillus subtilis* strain DSM 13182.

12. Drugs containing a galactomanno-oligosaccharide according to claim 7, optionally combined with a pharmaceutically compatible carrier.

13. Foods or luxury foodstuffs containing a galactomanno-oligosaccharide according to claim 7.

14. The use of a galactomanno-oligosaccharide according to claim 7 for producing foods and/or luxury foodstuffs and/or for reducing the glycemic index of foods and/or luxury foodstuffs.

15. The use of a galactomanno-oligosaccharide according to claim 7 in order to produce foods containing said galactomanno-saccharide for preventing infectious diseases, for preventing intestinal diseases, for preventing colon carcinogenesis, for strengthening the immune defence system against general infections, for preventing inflammatory diseases and/or for preventing osteoporosis.

16. The use of a galactomanno-oligosaccharide according to claim 7 in order to produce a drug for preventing infectious diseases, for preventing intestinal diseases, for preventing colon carcinogenesis, for strengthening the immune defence system against general infections, for preventing inflammatory diseases and/or for preventing osteoporosis.

## Revendications

1. Procédé pour la fabrication d'oligosaccharides de galactomannane contenant de la mannose et de la galactose, **caractérisé en ce qu'**une solution aqueuse ou une suspension du galactomannane est produite, puis hydrolysée en utilisant un agent enzymatique en bactéries du type Bacillus subtilis appartenant à la souche bactérienne enregistrée à la DSM (collection de cultures de microorganismes et cultures cellulaires d'Allemagne) sous le numéro 13182 pour obtenir ainsi une solution aqueuse d'un mélange d'oligosaccharides de galactomannane ayant un degré de polymérisation (DP) < 15.

2. Procédé selon la revendication 1, **caractérisé en ce que** le degré de polymérisation (DP) est de 2 à 7.

3. Procédé selon l'une des revendications 1 ou 2, **caractérisé en ce que** l'agent enzymatique sont des cellules bactériennes vivantes ou mortes.

4. Procédé selon l'une des revendications 1 ou 2, **caractérisé en ce que** l'agent enzymatique est une enzyme susceptible d'hydrolyser du galactomannane ou un extrait brut des cellules de ces bactéries.

5. Procédé selon l'une des revendications 1 à 4, **caractérisé en ce que** l'hydrolyse enzymatique est réalisée avec des cellules immobilisées, des extraits bruts immobilisés ou des enzymes immobilisées de ces bactéries.

6. Procédé selon l'une des revendications précédentes, **caractérisé en ce que** le mélange d'oligosaccharides de galactomannane ainsi reçu est soumis à un procédé de séparation chromatographique.

7. Oligosaccharide de galactomannane, susceptible d'être produit à partir de gomme de guar selon un procédé des revendications 1 à 6, comprenant des unités de mannose liées en β-1,4 enchaînées avec des unités de galactose liées en α-1,6 ayant un degré de polymérisation (DP) < 15, notamment compris entre 2 et 7.

8. Oligosaccharide de galactomannane, susceptible d'être produit à partir de gomme de guar selon un procédé des revendications 1 à 6, comprenant des unités de mannose liées en β-1,4 enchaînées avec des unités de galactose liées en α-1,6 ayant un degré de polymérisation < 15, notamment compris entre 2 et 7, pour une utilisation comme moyen thérapeutique ou de diagnostic.

9. Enzyme capable d'hydrolyser sans variations significatives d'efficacité des divers galactomannanes, de préférence de la gomme de guar, de la gomme de cassia et de la farine de graines de caroube, cette enzyme étant susceptible d'être produite à partir de la souche bactérienne Bacillus subtilis DSM 13182.

10. Extrait brut capable d'hydrolyser sans variations significatives d'efficacité des divers galactomannanes, de préférence de la gomme de guar, de la gomme de cassia et de la gomme de caroube, cet extrait brut étant susceptible d'être produit par fractionnement cellulaire des bactéries de la souche bactérienne Bacillus subtilis DSM 13182.

11. Souche bactérienne Bacillus subtilis DSM 13182.

12. Médicament contenant un oligosaccharide de galactomannane selon la revendication 7, le cas échéant avec un véhicule pharmaceutiquement acceptable.

13. Denrée alimentaire ou de luxe, contenant un oligosaccharide de galactomannane selon la revendication 7.

14. Utilisation d'un oligosaccharide de galactomannane selon la revendication 7 pour la fabrication de denrées de luxe et/ou de denrées alimentaires et/ou pour la réduction de l'index glycémique de denrées de luxes et/ou de denrées alimentaires.

15. Utilisation d'un oligosaccharide de galactomannane selon la revendication 7 pour la fabrication d'une denrée alimentaire contenant cet oligosaccharide de galactomannane pour éviter des maladies infectieuses, pour prévenir des maladies intestinales, pour prévenir la carcinogenèse du côlon, pour renforcer le système immunitaire contre des infections générales, pour prévenir des maladies inflammatoires et/ou pour prévenir l'ostéoporose.

16. Utilisation d'un oligosaccharide de galactomannane selon la revendication 7 pour la fabrication d'un médicament pour éviter des maladies infectieuses, pour prévenir des maladies intestinales, pour prévenir la carcinogenèse du côlon, pour renforcer le système immunitaire contre des infections générales, pour prévenir des maladies inflammatoires et/ou pour prévenir l'ostéoporose.
